# EUROPEAN PATENT SPECIFICATION

(11) **EP 4 612 647 B1**
(45) Date of publication and mention of the grant of the patent: **04.03.2026**
(21) Application number: 23790366.1
(22) Date of filing: 23.10.2023
(51) Int. Cl.: G06T 7/00

(54) **DEVICE, METHOD AND COMPUTER PROGRAM FOR SEGMENTING THE MEMBRANOUS SEPTUM**
VORRICHTUNG, VERFAHREN UND COMPUTERPROGRAMM ZUR SEGMENTIERUNG DES MEMBRANSEPTUMS
DISPOSITIF, PROCÉDÉ ET PROGRAMME INFORMATIQUE POUR SEGMENTER LE SEPTUM MEMBRANEUX

(30) Priority: 31.10.2022 EP 22204734
(43) Date of publication of application: 10.09.2025
(73) Proprietor: Koninklijke Philips N.V., 5656 AG Eindhoven (NL)
(72) Inventor: WEESE, Rolf Jürgen, 5656 AG Eindhoven (NL); WIEMKER, Rafael, 5656 AG Eindhoven (NL); BONTUS, Claas, 5656 AG Eindhoven (NL); PETERS, Jochen, 5656 AG Eindhoven (NL); BROSCH, Tom, 5656AG Eindhoven (NL); NICKISCH, Hannes, 5656 AG Eindhoven (NL)
(74) Representative: Philips Intellectual Property & Standards
(86) International application number: PCT/EP2023/079390
(87) International publication number: WO 2024/094449

(56) References cited:
- US-B1- 6 389 310
- TAKAYAMA HIROO ET AL: "Virtual septal myectomy for preoperative planning in hypertrophic cardiomyopathy", THE JOURNAL OF THORACIC AND CARDIOVASCULAR SURGERY, MOSBY-YEAR BOOK, INC., ST. LOUIS, MO, US, vol. 158, no. 2, 1 August 2019 (2019-08-01), pages 455 - 463, XP085734944, ISSN: 0022-5223, [retrieved on 20181114], DOI: 10.1016/J.JTCVS.2018.10.138

## Description

### FIELD OF THE INVENTION

The present invention relates to a device, method and computer program for segmenting the membranous septum.

### BACKGROUND OF THE INVENTION

The so-called membranous septum (i.e. the membranous, fibrous component of the overall cardiac septum) is a small structure in the heart that is located between the left ventricular outflow tract (LVOT) and the right ventricle / atrium and characterized by a thin wall between the left and the right heart chambers at the height of the tricuspid annulus. It indicates the location of conduction tracts in the heart. Certain geometric properties of the membranous septum are associated with the risk of needing a pacemaker after transthoracic aortic valve repair (TAVR).

The membranous septum is a small and unobtrusive structure on a non-planar surface that is difficult to find and annotate in 3D image data sets, e.g. cardiac computed tomography angiography (CTA) image data sets, in particular in standard planar slice views. In addition, anatomical knowledge is available that can help to identify the membranous septum when properly used. In that way, an extensive number of data sets together with tedious and thus costly manual annotations (as would be required for a neural network-based approach) shall be avoided.

A paper with the title *"*Virtual septal myectomy for preoparative planning in hyperhropic cardiomyopathy" by Takayama Hiroo et al published in The Journal of Thoracic and Cardiovascular Surgery, vol 158, no.2, 2019-08-01, pages 455-463, discloses a virtual myectomy (VM) technique using 3-dimensional reconstruction of gated cardiac computed tomography (CT) to assist intraoperative objective assessment of he adequacy of the resection.

### SUMMARY OF THE INVENTION

It is an object of the present invention to provide a device, method and computer program for efficiently and reliably segmenting the membranous septum in an automated manner.

In a first aspect of the present invention a device for segmenting the membranous septum is presented, the device comprising circuitry configured to:
- segment the left ventricular outflow tract, LVOT, of the heart in a 3D image data set;
- determine wall thickness information indicative of the wall thickness of the septum at different locations of a part of the segmented LVOT that is oriented towards the right heart chamber and right atrium;
- map the determined wall thickness information onto the surface of said part of the segmented LVOT; and
- segment the membranous septum in the 3D image data set based on the mapped wall thickness information.

In further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, computer program and medium have similar and/or identical preferred embodiments as the claimed system, in particular as defined in the dependent claims and as disclosed herein.

The present invention is based on the idea to enable an automated segmentation of the membranous septum by segmenting the LVOT and using the segmented LVOT to determine thickness information of the septum that is adjacent to the LVOT on the side of the right heart chamber and right atrium. Based on this thickness information the membranous septum can be found, in particular as the part of the overall cardiac septum having the smallest thickness. A mapping of the region of interest onto a two-dimensional reformat may simplify the subsequent image processing steps for manual and/or automatic delineation and quantification of the membranous septum.

According to an embodiment the circuitry is further configured to segment the membranous septum by applying any one of thresholding, independent component analysis, and k-nearest neighbor clustering of the surface of said part of the segmented LVOT. Generally, any standard approach for segmentation may be applied on the surface of the LVOT for this purpose.

The circuitry may further be configured to segment the LVOT using a model-based segmentation to adapt a heart model to the 3D image data set. This provides a good starting point for the subsequent steps of the processing.

In a preferred embodiment a heart model comprising an indication of an area in which the membranous septum should be found and/or comprising information allowing the generation of two or more planes perpendicular to the LVOT and/or the determination of the aortic valve annulus plane. This improves the segmentation of the membranous septum. Hereby, the information may e.g. include geometric information in the form of landmarks with coordinates, such as aortic valve centroid, aortic valve normal vector, annulus plane normal, ascending aorta normal vector, and/or septum centroid.

In a practical implementation the circuitry may further be configured to determine the wall thickness information by
- determining an axis approximating the centerline of the LVOT;
- defining a set of rays that are perpendicular to said axis and/said surface and pass the wall of the LVOT in said part of the segmented LVOT;
- determining, for the rays of said set, corresponding wall thickness information; and
- mapping the wall thickness information on the location of the surface at which the respective ray passes the wall of the LVOT.

Hereby, the circuitry may further be configured to determine the axis by
- determining the aortic valve annulus plane before determining the wall thickness information; and
- defining an axis perpendicular to the aortic valve annulus plane.

In another implementation the circuitry may further be configured to determine the axis by fitting a line through the centroids of two or more surface rings of the ascending aorta, in particular as provided by an adapted geometric model (model based segmentation).

Still further, in another implementation, the circuitry may further be configured to determine, as wall thickness information, a wall thickness value of the thickness of the septum along the respective ray or a surrogate value of the wall thickness value.

Hereby, as surrogate value of the wall thickness value, an average image value (or pixel value or gray value) of a ray segment of the respective ray that starts at the surface of the LVOT and has a predetermined or adaptable length. The image value is related to the X-ray opacity (radio-opacity, typically in Hounsfield units, HU), which in turn is correlated (approximately linear) with the mass density (in relation to water and air). Therefore, for an assumed constant material, the accumulated densities yield an approximate mass, which translates into a length (thickness).

Further, the wall thickness value may be determined based on the length of a ray segment of the respective ray that starts at the surface of the LVOT and ends at a change of the image value (or pixel value or gray value) indicating a change of tissue.

According to another embodiment the wall thickness information may be determined by use of a trained algorithm or computer system, in particular trained machine learning, a trained classifier or a trained neural network, that has been trained to estimate the wall thickness of organic structures in 3D image data sets of the heart. This avoids the need to apply a dedicated algorithm but allows to use artificial intelligence for this task.

The circuitry may further be configured to determine the axis by computing a distance map inside the LVOT and maximizing the distance of a straight line in the LVOT to the surface of the LVOT.

According to another embodiment the circuitry is further configured to determine an area in which the membranous septum should be found by
- segmenting the right ventricle and right atrium;
- finding the point that is closest to the LVOT;
- identifying the ray passing this point; and
- defining an angular range for rotating the ray around the axis and a translational range for moving the ray up or down the axis.

According to still another embodiment the circuitry is further configured to
- utilize a geometric model represented by a triangle mesh to identify, for a respective ray outward from the LVOT manifold surface, the 3D coordinate where the ray intersects with one of the mesh triangles intersecting triangle vertices; and
- use the 3D coordinate as the starting point for a ray that estimates the local thickness of the septum.

The circuitry may further be configured to visualize the determined wall thickness information and/or the segmented membranous septum on the surface of said part of the segmented LVOT or on a flattened reformat of said part of the segmented LVOT. This helps the user to understand the result of the segmentation and to make a diagnosis.

### BRIEF DESCRIPTION OF THE DRAWINGS

These and other aspects of the invention will be apparent from and elucidated with reference to the embodiment(s) described hereinafter. In the following drawings
Fig. 1 shows a schematic drawing of a part of the anatomy of the human heart;
Fig. 2 shows a schematic diagram of an embodiment of a device according to the present invention;
Fig. 3 shows a flow chart of an embodiment of a method according to the present invention;
Fig. 4 shows a flow chart of another embodiment of a method according to the present invention;
Fig. 5 shows a schematic drawing of a heart model and of a left ventricle into which additional information is encoded;
Fig. 6 shows two cross-sectional views through the heart along different planes.
Fig. 7 shows a cross-sectional view to indicate the search space in z-direction.
Fig. 8 shows cross-sectional view from a different perspective.
Fig. 9 shows cross-sectional views to indicate the search space in rotational direction.
Fig. 10 illustrates the result of an MBS-based implementation giving a manifold / map draped as texture.
Fig. 11 illustrates the manifold / map with the footprint of right ventricle and right atrium.
Fig. 12 shows diagrams illustrating another exemplary implementation of the present invention.

### DETAILED DESCRIPTION OF EMBODIMENTS

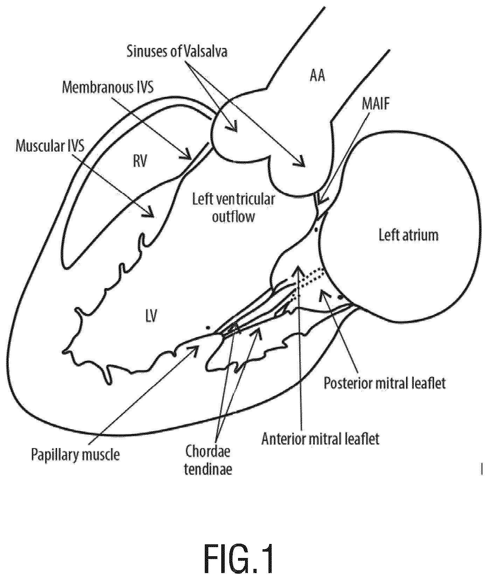

Fig. 1 shows a schematic drawing of major parts of the anatomy of the human heart. The left ventricular outflow (LVO) is an anatomically complex region of the left ventricle (LV), which gives rise to the ascending aorta (AA), provides support to the aortic valvular cusps, and houses important components of the conduction system. The LV is situated between the anterior leaflet of the mitral valve (MV) and the left ventricular smooth side of the muscular and membranous interventricular septum (IVS). The anterior leaflet of the MV (posterolateral wall) is displaced from the IVS by the LVO. The region of the fibrous continuity is referred to as the mitral aortic intervalvular fibrosa (MAIF).

The membranous septum (membranous IVS) is a small compact fibrous part of the IVS that lies between the non-coronary and right coronary aortic cusp and the septal leaflet of the tricuspid valve (TV) and therefore, at its upper end, is continuous with the right lateral wall of the aortic root. Also, the membranous septum is partly atrio-ventricular and anatomically separates a small portion of the right atrium nearest the septal leaflet of the TV from the LV. At the junction of the muscular and membranous IVS lies the atrioventricular (AV) bundle.

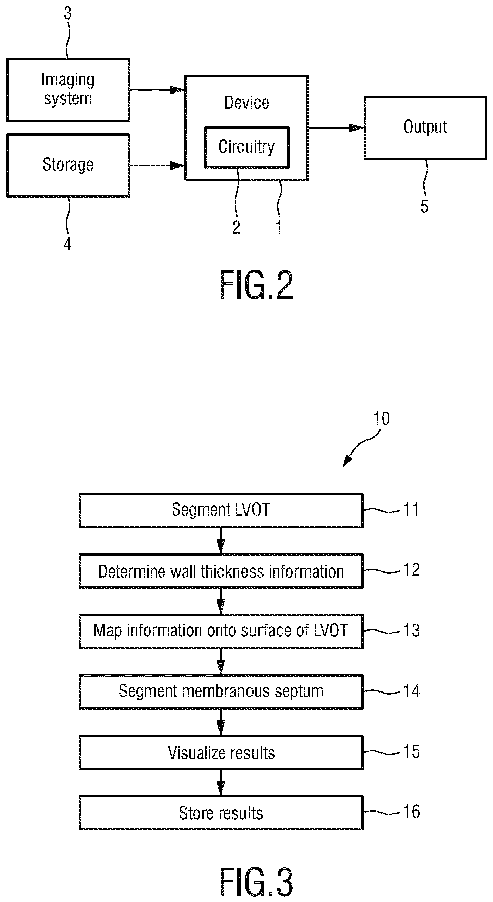

Fig. 2 shows a schematic diagram of an embodiment of a device 1 for segmenting the membranous septum according to the present invention. The device 1 comprises circuitry 2, e.g. a processor or computer, configured to carry out the steps of a method 10 for segmenting the membranous septum, an embodiment of which being schematically illustrated in Fig. 3.

A first step 11 of the method 10 segments the left ventricular outflow tract (LVOT) of the heart in a 3D image data set, which may be directly obtained from an imaging system 3 (e.g. a CT imaging system, an X-ray system, etc.) or from an image storage or repository 4 (e.g. an image archive or database of a hospital). The 3D image data set may e.g. be a cardiac computed tomography angiography (CTA) image data set acquired from a patient, which may be processed on the fly for providing important information to a physician, e.g. live during a surgery or for making a diagnosis.

A second step 12 of the method determines wall thickness information indicative of the wall thickness of the septum at different locations of a part of the segmented LVOT that is oriented towards the right heart chamber and right atrium. As explained above, the thickness of the septum changes along its longitudinal extension so that the wall thickness information is useful to identify the membranous septum.

A third step 13 maps the determined wall thickness information onto the surface of said part of the segmented LVOT, i.e., the part of the segmented LVOT that is oriented towards the right heart chamber and right atrium as used in step 12.

A fourth step 14 segments the membranous septum in the 3D image data set based on the mapped wall thickness information. The region of the membranous septum may hereby be segmented by thresholding, independent component analysis, k-nearest neighbor (k-NN) clustering or another approach on the surface of the LVOT.

A fifth (optional) step 15 may visualize, e.g. display on a screen 5, the wall thickness information and/or the segmentation result (the segmented membranous septum) on the surface of the LVOT or an appropriately flattened reformat. Further, tools for interactive correction of the segmentation by the user may be offered, e.g., as a user interface.

A sixth (optional) step 16 may store the wall thickness information and/or the segmentation result, e.g. together with the 3D image data set or with information indicating that it belongs to the 3D image data set. For instance, it may be stored as annotation of the 3D image data set.

In an embodiment, the device may be implemented with dedicated units or means, such as an input unit for obtaining the 3D image data set, a processing unit for carrying out the steps of the method 10, and an output unit for outputting the results.

The input unit may be directly coupled or connected to the imaging system or may obtain (i.e. retrieve or receive) these signals from a storage, buffer, network, or bus, etc. The input unit may thus e.g. be (wired or wireless) communication interfaces or data interfaces, such as a Bluetooth interface, Wi-Fi interface, LAN interface, HDMI interface, direct cable connect, or any other suitable interface allowing signal transfer to the device.

The processing unit may be configured perform the steps of the method 10 and may be any kind of means configured to process the 3D image data set. It may be implemented in software and/or hardware, e.g. as a programmed processor or computer or app on a user device.

The output unit may generally be any interface that provides the determined results, e.g. transmits it to another device or provides it for retrieval by another device (e.g. a computer, tablet, hospital network, etc.). It may thus generally be any (wired or wireless) communication or data interface.

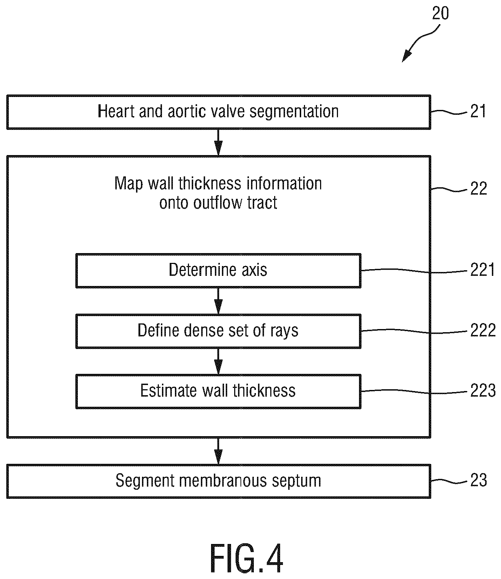

In the following a more detailed embodiment of the method 20 according to the present invention using model-based segmentation (MBS) will be explained as illustrated in the flow chart shown in Fig. 4.

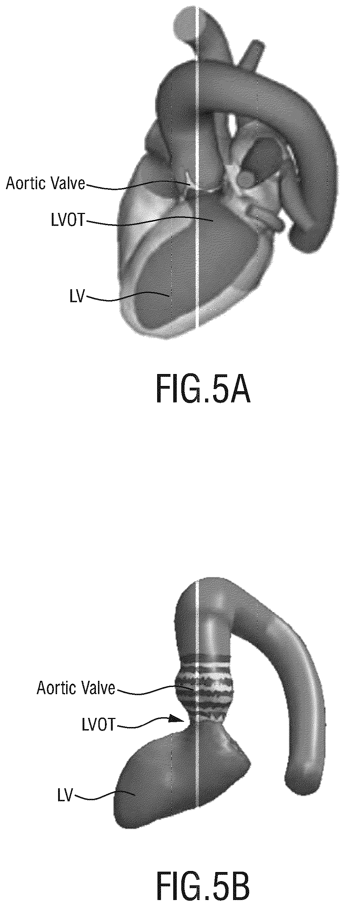

As a first step 21 of the segmentation approach, a heart and aortic valve segmentation is done (which generally includes step 11 above that segments the LVOT), for instance using the model-based method described in Waechter I. et al. "Patient Specific Models for Planning and Guidance of Minimally Invasive Aortic Valve Implantation" in "Medical Image Computing and Computer-Assisted Intervention - MICCAI 2010", Lecture Notes in Computer Science 2010, vol 6361, 526 - 533. This approach uses a model-based segmentation to adapt a heart model including a detailed model of the aortic valve, as shown in Fig. 5A (taken from this disclosure), to an image. In addition, information is encoded in the model, as shown in Fig. 5B (taken from this disclosure as well), that allows, for instance, to construct planes perpendicular to the outflow tract or to determine the aortic valve annulus plane. The information encoded in the model is complemented by information about the area where the membranous septum can be found (e.g. using information from publications or by using the region where the membranous septum has been found in previous cases enlarged by a safety margin).

In a second step 22 (which is substantially identical to step 13), wall thickness information is mapped onto the outflow tract where the membranous septum is expected to be. This may be done by the following sub-steps.

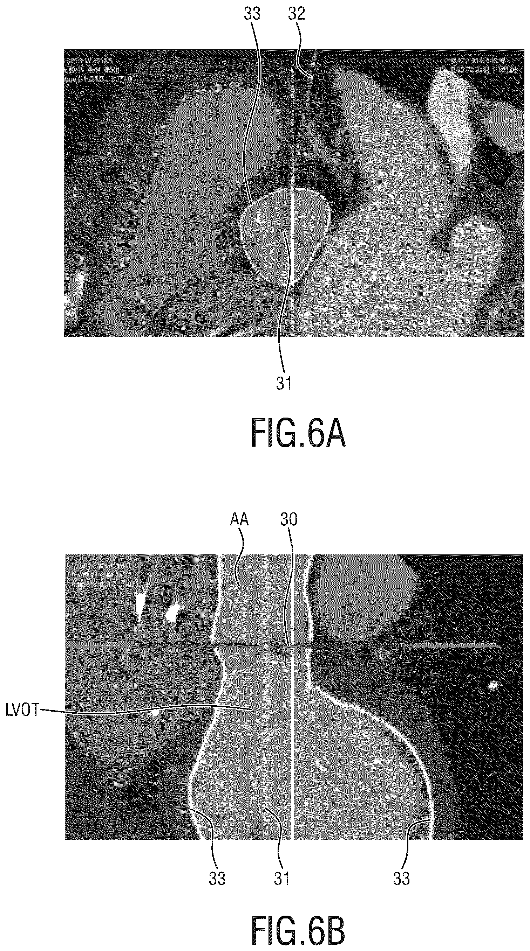

In a first sub-step 221, an axis approximating the centerline of the outflow tract is determined. This can be done by determining the aortic valve annulus plane 30 (see Fig. 6 showing sectional views through the AA and the LVOT) and defining an axis 31 perpendicular to it in the middle of the region where the annulus plane 30 intersects with the outflow tract LVOT. Alternatively, an axis can be defined by fitting a line through the centroids of two or more surface rings of the ascending aorta, in particular as provided by an adapted geometric mode. Fig. 6A shows a cross-sectional view through the annulus plane 30 shown in Fig. 6B. Fig. 6B shows a cross-sectional view through another plane 32 (a long-axis-plane with respect to the aortic valve (as opposed to a long-axis-plane for the left ventricle) that is perpendicular to the annulus plane 30 and goes through the axis 31. Line 33 indicates the wall of the AA (in Figs. 6A and 6B) and the LV and LVOT (in Fig. 6B).

In a second sub-step 222, the axis 31 is used to define a dense set of rays (one ray being arranged in the other plane 32 in Fig. 6A) that are perpendicular to the axis 31 and pass the wall 33 of the outflow tract in the region where the membranous septum can be found according to a label encoded on the heart model. For each of the rays, a quantity characteristic of the septal wall thickness (a surrogate measure) is computed and mapped onto the mesh surface.

In a third sub-step 223, an example of such a quantity is, for instance, the average gray value (or pixel value or image value) of a ray segment that starts at the surface of the LVOT and has a given length. This average gray value will be smaller the more uncontrasted tissue is in between the contrasted chambers. Alternatively, machine learning may be used to train classifiers that classify a profile into belonging to the septum or not or to train a regression network that estimates the wall thickness.

In the third step 23, the region of the membranous septum is segmented, which may be done by thresholding, independent component analysis, k-NN clustering or another approach using the quantity that is characteristic for the wall thickness as feature. Visualization of the segmentation result and interactive correction (e.g. by editing a contour on the curved surface after proper conversion of the segmentation result) may be done with standard methods, for instance by electronic pencils, brushes, etc., to draw or delineate and modify 2D contours on a two-dimensional area.

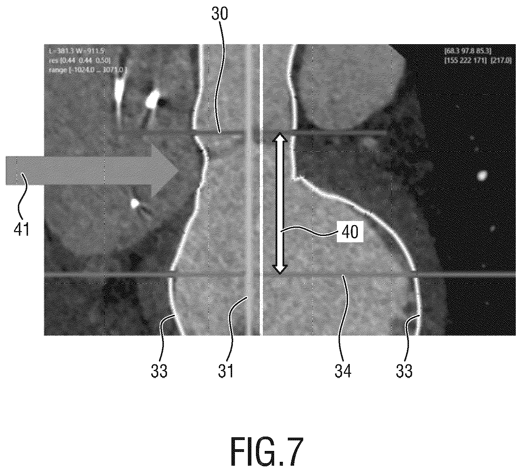

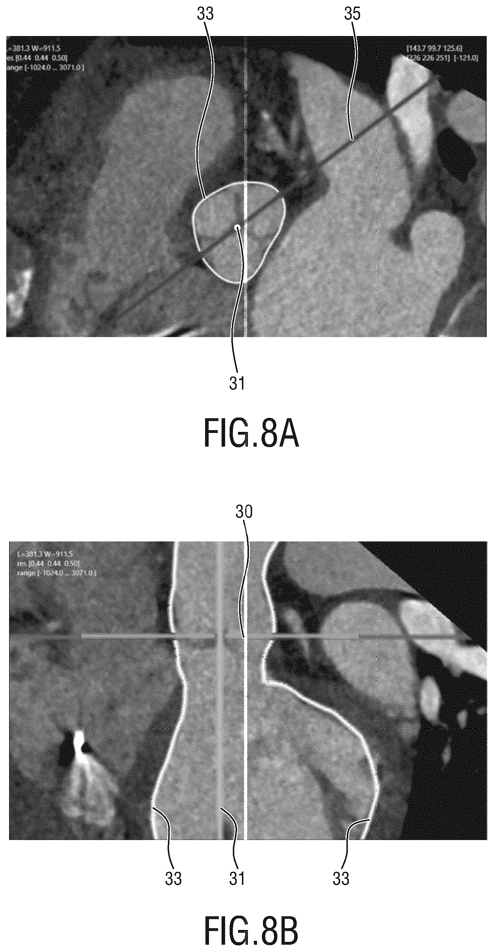

Figs. 7 to 11 illustrate the approach and the results. Fig. 7 shows a cross-sectional view similar to the cross-sectional view shown in Fig. 6B. It indicates the search space in z-direction (indicated by arrow 40) along the axis 31 from the annulus plane 30 to an LV plane 34. The arrow 41 indicates the approximate position of the membranous septum. Fig. 8 shows similar cross-sectional views as shown in Fig. 6, but where the cross-sectional view 8B is shown for a different plane 35 that is rotated around the axis compared to the plane 32 shown in Fig. 6A.

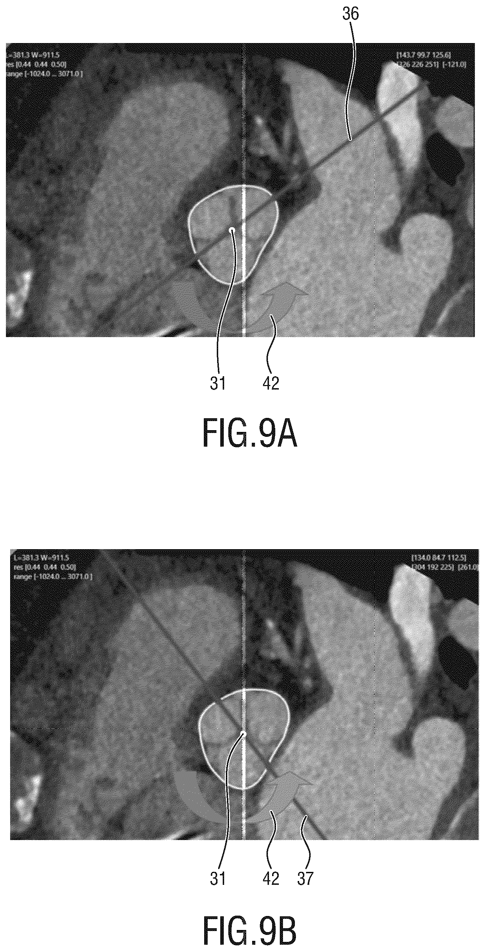

Fig. 9 shows cross-sectional views similar to the cross-sectional view shown in Fig. 6a to indicate the how the ray (indicated as ray 36 in Fig. 9A and ray 37 in Fig. 9B) rotates around the axis 31. This is the search space in angular direction (indicated by arrow 42).

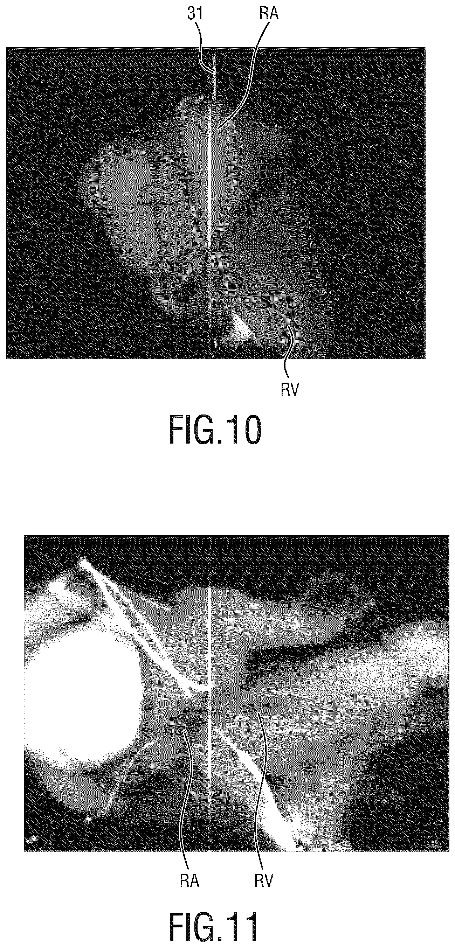

Fig. 10 illustrates the result of an MBS-based implementation giving a manifold / map draped as texture over a model mesh surface with translucent RV and RA. Fig. 11 illustrates the manifold / map with the footprint of RV and RA.

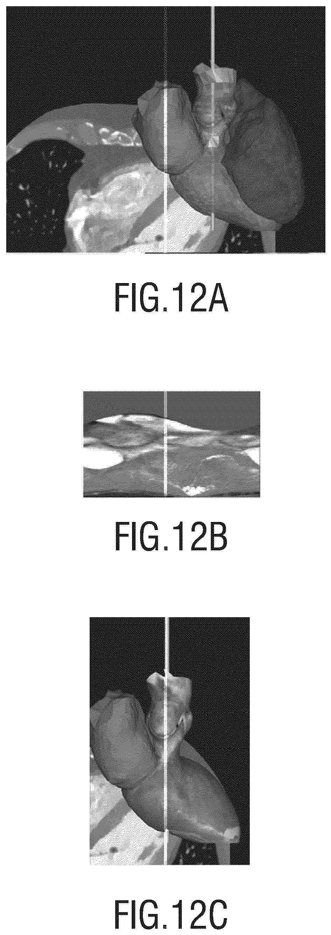

Fig. 12 shows diagrams illustrating another exemplary implementation of the present invention. As shown in Fig. 12A, from a centroid of a mesh triangle of a mesh modeling part of the heart anatomy (including the right ventricle), a ray is sampled in orthogonal direction to the LVOT axis 31. The ray may have a length in the range of 5 to 10 mm, e.g. 6 or 10 mm length, wherein a predetermined value may be used and set to a value that covers the thin part of the thickness of the septum. The ray's starting point for the rays is generally on the LVOT/aortic surface. The ray's mean density (HU) is mapped to the rendering gray-value color (image value or pixel value) of the triangle mesh. Thus, e.g. light gray may indicate a thin low density part of the septum and dark gray may indicate a thick low density part of the septum.

Fig. 12B shows a flattened (i.e. curvilinear reformatted) two-dimensional manifold, which is parametrized by a cylindrical mapping of the LVOT and aortic mesh surface, using height and angle with respect to the LVOT axis. The sampling rays are not originating from the sparse triangles' centroids, but rather sampled in a dense fashion from the flattened two-dimensional manifold.

Fig. 12C show a diagram in which the right ventricle is hidden for improved visual inspection in order not to occlude the septum. The densely sampled two-dimensional manifold shown in Fig. 12B is used for a texture mapping and folded around the triangle mesh representation of the LVOT and aortic surface, resulting in a finer and thus clearer representation.

Instead of using model-based segmentation in step 21, the outflow tract can also be segmented as a voxel mask using neural networks. The axis used in sub-step 221 to cast the rays can then be determined by computing a distance map inside the outflow tract and maximizing the distance of a straight line in the outflow tract to the surface of the outflow tract. The rough area, where the membranous septum can be found may be defined by segmenting the right ventricle and right atrium, finding the point that is closest to the LVOT, identifying the ray passing this point and defining an angular range for rotating the ray around the axis and a translational rage for moving the ray up or down the axis. Identification of the membranous septum by casting rays and computing quantities indicative of the wall thickness can be done as described for the implementation example using MBS in sub-steps 222 and 223.

Two-dimensional manifold construction from triangle mesh and LVOT axis may be performed as follows. For each cylindrical (z, Φ)-coordinate on the LVOT axis fit, an axis-orthogonal ray may be cast out from the axis, and the closest intersecting triangle vertices may be identified (implemented e.g. as an efficient KD (K-dimensional) tree search). The 3D-coordinate of the respective manifold surface point may then be determined e.g. as the mean of the k-nearest-mesh-vertex-neighbors. Thus, all landmarks, which can aid to define the membranous septum, can be interrelated in all three representations (image volume, surface mesh, reformat manifold), both for manual annotation as well as for automatic algorithmic purposes.

In summary, the present invention allows to efficiently and reliably segment the membranous septum in an automated manner. The disclosed approach can be used with other types of images such as MR or spectral CT images. Spectral CT images may be used to better resolve the material properties of the underlying tissue.

The segmentation result can be used to visualize the membranous septum and to extract measurements such as the distance between the aortic valve plane and the lower and/or upper rim of the septum in a transcatheter aortic valve replacement (TAVR) application, for instance, in a medical diagnostic view system such as the Philips Intellispace Portal.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (1) for segmenting the membranous septum in a 3D image data set, the device comprising circuitry (2) configured to:
- segment the left ventricular outflow tract, LVOT, of the heart in the 3D image data set;
- determine wall thickness information indicative of the wall thickness of the septum at different locations of a part of the segmented LVOT that is oriented towards the right heart chamber and right atrium;
- map the determined wall thickness information onto the surface of said part of the segmented LVOT; and
- segment the membranous septum in the 3D image data set based on the mapped wall thickness information.

2. Device (1) as claimed in claim 1,
wherein the circuitry (2) is further configured to segment the membranous septum by applying any one of thresholding, independent component analysis, and k-nearest neighbor clustering of the surface of said part of the segmented LVOT.

3. Device (1) as claimed in any one of the preceding claims,
wherein the circuitry (2) is further configured to segment the LVOT using a model-based segmentation to adapt a heart model to the 3D image data set, in particular to use a heart model comprising an indication of an area in which the membranous septum should be found and/or comprising information allowing the generation of two or more planes perpendicular to the LVOT and/or the determination of the aortic valve annulus plane.

4. Device (1) as claimed in any one of the preceding claims,
wherein the circuitry (2) is further configured to determine the wall thickness information by
- determining an axis approximating the centerline of the LVOT;
- defining a set of rays that are perpendicular to said axis and/said surface and pass the wall of the LVOT in said part of the segmented LVOT;
- determining, for the rays of said set, corresponding wall thickness information; and
- mapping the wall thickness information on the location of the surface at which the respective ray passes the wall of the LVOT.

5. Device (1) as claimed in claim 4,
wherein the circuitry (2) is further configured to determine the axis by
- determining the aortic valve annulus plane before determining the wall thickness information; and
- defining an axis perpendicular to the aortic valve annulus plane.

6. Device (1) as claimed in claim 4,
wherein the circuitry (2) is further configured to determine the axis by fitting a line through the centroids of two or more surface rings of the ascending aorta, in particular as provided by an adapted geometric model.

7. Device (1) as claimed in claim 4,
wherein the circuitry (2) is further configured to determine, as wall thickness information, a wall thickness value of the thickness of the septum along the respective ray or a surrogate value of the wall thickness value.

8. Device (1) as claimed in claim 7,
wherein the circuitry (2) is further configured to determine, as surrogate value of the wall thickness value, an average image value of a ray segment of the respective ray that starts at the surface of the LVOT and has a predetermined or adaptable length, and/or to determine the wall thickness value based on the length of a ray segment of the respective ray that starts at the surface of the LVOT and ends at a change of the image value indicating a change of tissue.

9. Device (1) as claimed in claim 7,
wherein the circuitry (2) is further configured to determine the wall thickness information by use of a trained algorithm or computer system, in particular trained machine learning, a trained classifier or a trained neural network, that has been trained to estimate the wall thickness of organic structures in 3D image data sets of the heart.

10. Device (1) as claimed in claim 4,
wherein the circuitry (2) is further configured to determine the axis by computing a distance map inside the LVOT and maximizing the distance of a straight line in the LVOT to the surface of the LVOT.

11. Device (1) as claimed in claim 4,
wherein the circuitry (2) is further configured to determine an area in which the membranous septum should be found by
- segmenting the right ventricle and right atrium;
- finding the point that is closest to the LVOT;
- identifying the ray passing this point; and
- defining an angular range for rotating the ray around the axis and a translational range for moving the ray up or down the axis.

12. Device (1) as claimed in claim 4,
wherein the circuitry (2) is further configured to
- utilize a geometric model represented by a triangle mesh to identify, for a respective ray outward from the LVOT manifold surface, the 3D coordinate where the ray intersects with one of the mesh triangles intersecting triangle vertices; and
- use the 3D coordinate as the starting point for a ray that estimates the local thickness of the septum.

13. Device (1) as claimed in any one of the preceding claims,
wherein the circuitry (2) is further configured to visualize the determined wall thickness information and/or the segmented membranous septum on the surface of said part of the segmented LVOT or on a flattened reformat of said part of the segmented LVOT.

14. Computer implemented method (10) for segmenting the membranous septum in a 3D image data set, the method comprising:
- segmenting (11) the left ventricular outflow tract, LVOT, of the heart in the 3D image data set;
- determining (12) wall thickness information indicative of the wall thickness of the septum at different locations of a part of the segmented LVOT that is oriented towards the right heart chamber and right atrium;
- mapping (13) the determined wall thickness information onto the surface of said part of the segmented LVOT; and
- segmenting (14) the membranous septum in the 3D image data set based on the mapped wall thickness information.

15. Computer program comprising program code means for causing a computer to carry out the steps of the method as claimed in claim 14 when said computer program is carried out on the computer.

## Patentansprüche

1. Vorrichtung (1) zur Segmentierung des membranösen Septums in einem 3D-Bilddatensatz, wobei die Vorrichtung eine Schaltung (2) umfasst, welche dazu konfiguriert ist:
- den linksventrikulären Ausflusstrakt, LVOT, des Herzens in dem 3D-Bilddatensatz zu segmentieren;
- Wanddickeninformationen zu bestimmen, welche die Wanddicke des Septums an unterschiedlichen Stellen eines Teils des segmentierten LVOT angeben, welcher zur rechten Herzkammer und zum rechten Vorhof hin ausgerichtet ist;
- die bestimmten Wanddickeninformationen auf der Oberfläche des Teils des segmentierten LVOT abzubilden; und
- das membranöse Septum in dem 3D-Bilddatensatz basierend auf den abgebildeten Wanddickeninformationen zu segmentieren.

2. Vorrichtung (1) nach Anspruch 1,
wobei die Schaltung (2) weiter dazu konfiguriert ist, das membranöse Septum zu segmentieren, indem sie eines von Schwellenwertbildung, unabhängiger Komponentenanalyse oder k-nächster-Nachbarn-Clustering der Oberfläche des segmentierten Teils des LVOT anwendet.

3. Vorrichtung (1) nach einem der vorstehenden Ansprüche,
wobei die Schaltung (2) weiter dazu konfiguriert ist, den LVOT unter Verwendung einer modellbasierten Segmentierung zu segmentieren, um ein Herzmodell an den 3D-Bilddatensatz anzupassen, insbesondere um ein Herzmodell zu verwenden, welches eine Angabe eines Bereichs umfasst, in welchem sich das membranöse Septum befinden sollte, und/oder Informationen umfasst, welche die Erzeugung von zwei oder mehr Ebenen senkrecht zu dem LVOT und/oder die Bestimmung der Ebene des Aortenklappenanulus ermöglichen.

4. Vorrichtung (1) nach einem der vorstehenden Ansprüche,
wobei die Schaltung (2) weiter dazu konfiguriert ist, die Wanddickeninformationen wie folgt zu bestimmen:
- Bestimmen einer Achse, welche sich der Mittellinie des LVOT annähert;
- Definieren eines Satzes von Strahlen, welche senkrecht zu der Achse und/der Oberfläche stehen und die Wand des LVOT in dem Teil des segmentierten LVOT passieren;
- Bestimmen der entsprechenden Wanddickeninformationen für die Strahlen des Satzes; und
- Abbilden der Wanddickeninformationen an der Stelle der Oberfläche, an welcher der jeweilige Strahl die Wand des LVOT passiert.

5. Vorrichtung (1) nach Anspruch 4,
wobei die Schaltung (2) weiter dazu konfiguriert ist, die Achse wie folgt zu bestimmen:
- Bestimmen der Ebene des Aortenklappenanulus vor Bestimmen der Wanddickeninformationen; und
- Definieren einer Achse, welche senkrecht zu der Ebene des Aortenklappenanulus verläuft.

6. Vorrichtung (1) nach Anspruch 4,
wobei die Schaltung (2) weiter dazu konfiguriert ist, die Achse durch Einpassen einer Linie durch die Schwerpunkte von zwei oder mehr Oberflächenringen der Aorta ascendens zu bestimmen, insbesondere wie sie von einem adaptierten geometrischen Modell bereitgestellt wird.

7. Vorrichtung (1) nach Anspruch 4,
wobei die Schaltung (2) weiter dazu konfiguriert ist, als Wanddickeninformationen einen Wanddickenwert der Dicke des Septums entlang des jeweiligen Strahls oder einen Ersatzwert des Wanddickenwerts zu bestimmen.

8. Vorrichtung (1) nach Anspruch 7,
wobei die Schaltung (2) weiter dazu konfiguriert ist, als Ersatzwert für den Wanddickenwert einen durchschnittlichen Bildwert eines Strahlsegments des jeweiligen Strahls zu bestimmen, welcher an der Oberfläche des LVOT beginnt und eine vorbestimmte oder anpassbare Länge aufweist, und/oder den Wanddickenwert basierend auf der Länge eines Strahlsegments des jeweiligen Strahls zu bestimmen, welcher an der Oberfläche des LVOT beginnt und an einer Änderung des Bildwerts endet, welche eine Änderung des Gewebes angibt.

9. Vorrichtung (1) nach Anspruch 7,
wobei die Schaltung (2) weiter dazu konfiguriert ist, die Wanddickeninformationen durch Verwendung eines trainierten Algorithmus oder Computersystems, insbesondere eines trainierten maschinellen Lernens, eines trainierten Klassifikators oder eines trainierten neuronalen Netzes, zu bestimmen, welche darauf trainiert wurden, die Wanddicke organischer Strukturen in 3D-Bilddatensätzen des Herzens zu schätzen.

10. Vorrichtung (1) nach Anspruch 4,
wobei die Schaltung (2) weiter dazu konfiguriert ist, die Achse durch Berechnen einer Abstandskarte innerhalb des LVOT zu bestimmen und den Abstand einer geraden Linie in dem LVOT zu der Oberfläche des LVOT zu maximieren.

11. Vorrichtung (1) nach Anspruch 4,
wobei die Schaltung (2) weiter dazu konfiguriert ist, einen Bereich, in welchem das membranöse Septum gefunden werden soll, wie folgt zu bestimmen:
- Segmentieren des rechten Ventrikels und des rechten Vorhofs;
- Finden des Punkts, welcher dem LVOT am nächsten liegt;
- Identifizieren des Strahls, welcher diesen Punkt passiert; und
- Definieren eines Winkelbereichs zum Drehen des Strahls um die Achse und eines Translationsbereichs zum Bewegen des Strahls entlang der Achse nach oben oder unten.

12. Vorrichtung (1) nach Anspruch 4,
wobei die Schaltung (2) weiter dazu konfiguriert ist,
- ein geometrisches Modell, dargestellt durch ein Dreiecksnetz zur Identifikation, zu nutzen, um für einen jeweiligen Strahl, welcher von der LVOT-Verteileroberfläche nach außen verläuft, die 3D-Koordinate zu identifizieren, an welcher der Strahl einen der sich schneidenden Dreiecksecken des Netzes schneidet; und
- die 3D-Koordinate als Ausgangspunkt für einen Strahl zu verwenden, welcher die lokale Dicke des Septums schätzt.

13. Vorrichtung (1) nach einem der vorstehenden Ansprüche,
wobei die Schaltung (2) weiter dazu konfiguriert ist, die bestimmten Wanddickeninformationen und/oder das segmentierte membranöse Septum auf der Oberfläche des Teils des segmentierten LVOT oder auf einer abgeflachten Umgestaltung des Teils des segmentierten LVOT zu visualisieren.

14. Computerimplementiertes Verfahren (10) zur Segmentierung des membranösen Septums in einem 3D-Bilddatensatz, wobei das Verfahren umfasst:
- Segmentieren (11) des linksventrikulären Ausflusstrakts, LVOT, des Herzens in dem 3D-Bilddatensatz;
- Bestimmen (12) von Wanddickeninformationen, welche die Wanddicke des Septums an unterschiedlichen Stellen eines Teils des segmentierten LVOT angeben, welcher zur rechten Herzkammer und zum rechten Vorhof hin ausgerichtet ist;
- Abbilden (13) der bestimmten Wanddickeninformationen auf der Oberfläche des Teils des segmentierten LVOT; und
- Segmentieren des membranösen Septums in dem 3D-Bilddatensatz basierend auf den abgebildeten Wanddickeninformationen.

15. Computerprogramm, das Programmcodemittel umfasst zum Bringen eines Computers dazu, die Schritte des Verfahrens nach Anspruch 14 auszuführen, wenn das Computerprogramm auf dem Computer ausgeführt wird.

## Revendications

1. Dispositif (1) pour segmenter le septum membraneux dans un ensemble de données d'image 3D, le dispositif comprenant une circuiterie (2) configurée pour :
- segmenter la cavité de chasse du ventricule gauche, LVOT, du cœur dans l'ensemble de données d'image 3D ;
- déterminer des informations d'épaisseur de paroi indiquant l'épaisseur de paroi du septum à différents endroits d'une partie de la LVOT segmentée qui est orientée vers la cavité cardiaque droite et l'oreillette droite ;
- reporter les informations d'épaisseur de paroi déterminées à la surface de ladite partie de la LVOT segmentée ; et
- segmenter le septum membraneux dans l'ensemble de données d'image 3D sur la base des informations d'épaisseur de paroi reportées.

2. Dispositif (1) selon la revendication 1,
dans lequel la circuiterie (2) est en outre configurée pour segmenter le septum membraneux en appliquant l'un quelconque parmi le seuillage, l'analyse en composantes indépendantes et le regroupement des k plus proches voisins de la surface de ladite partie de la LVOT segmentée.

3. Dispositif (1) selon l'une quelconque des revendications précédentes,
dans lequel la circuiterie (2) est en outre configurée pour segmenter la LVOT à l'aide d'une segmentation basée sur un modèle afin d'adapter un modèle cardiaque à l'ensemble de données d'image 3D, en particulier pour utiliser un modèle cardiaque comprenant une indication d'une zone où devrait se trouver le septum membraneux et/ou comprenant des informations permettant la génération de deux plans ou plus perpendiculaires à la LVOT et/ou la détermination du plan d'anneau valvulaire aortique.

4. Dispositif (1) selon l'une quelconque des revendications précédentes,
dans lequel la circuiterie (2) est en outre configurée pour déterminer les informations d'épaisseur de paroi par
- la détermination d'un axe approximant la ligne médiane de la LVOT ;
- la définition d'un ensemble de rayons qui sont perpendiculaires audit axe et/ou à ladite surface et franchissent la paroi de la LVOT dans ladite partie de la LVOT segmentée ;
- la détermination, pour les rayons dudit ensemble, d'informations d'épaisseur de paroi correspondantes ; et
- le report des informations d'épaisseur de paroi à l'emplacement de la surface où le rayon respectif franchit la paroi de la LVOT.

5. Dispositif (1) selon la revendication 4,
dans lequel la circuiterie (2) est en outre configurée pour déterminer l'axe par
- la détermination du plan d'anneau valvulaire aortique avant la détermination des informations d'épaisseur de paroi ; et
- la définition d'un axe perpendiculaire au plan d'anneau valvulaire aortique.

6. Dispositif (1) selon la revendication 4,
dans lequel la circuiterie (2) est en outre configurée pour déterminer l'axe en ajustant une ligne à travers les centroïdes de deux anneaux de surface ou plus de l'aorte ascendante, notamment comme prévu par un modèle géométrique adapté.

7. Dispositif (1) selon la revendication 4,
dans lequel la circuiterie (2) est en outre configurée pour déterminer, comme informations d'épaisseur de paroi, une valeur d'épaisseur de paroi de l'épaisseur du septum le long du rayon respectif ou une valeur de substitution de la valeur d'épaisseur de paroi.

8. Dispositif (1) selon la revendication 7,
dans lequel la circuiterie (2) est en outre configurée pour déterminer, comme valeur de substitution de la valeur d'épaisseur de paroi, une valeur d'image moyenne d'un segment de rayon du rayon respectif qui commence à la surface de la LVOT et présente une longueur prédéterminée ou adaptable, et/ou pour déterminer la valeur d'épaisseur de paroi sur la base de la longueur d'un segment de rayon du rayon respectif qui commence à la surface de la LVOT et se termine à un changement de la valeur d'image indiquant un changement de tissu.

9. Dispositif (1) selon la revendication 7,
dans lequel la circuiterie (2) est en outre configurée pour déterminer les informations d'épaisseur de paroi à l'aide d'un algorithme ou d'un système informatique entraîné, en particulier un apprentissage automatique entraîné, un classificateur entraîné ou un réseau neuronal entraîné, qui a été entraîné à estimer l'épaisseur de paroi de structures organiques dans des ensembles de données d'image 3D du cœur.

10. Dispositif (1) selon la revendication 4,
dans lequel la circuiterie (2) est en outre configurée pour déterminer l'axe en calculant une carte de distance à l'intérieur de la LVOT et en maximisant la distance d'une ligne droite dans la LVOT à la surface de la LVOT.

11. Dispositif (1) selon la revendication 4,
dans lequel la circuiterie (2) est en outre configurée pour déterminer une zone dans laquelle le septum membraneux doit être trouvé par
- la segmentation du ventricule droit et de l'oreillette droite ;
- la découverte du point le plus proche de la LVOT ;
- l'identification du rayon passant par ce point ; et
- la définition d'une plage angulaire pour faire tourner le rayon autour de l'axe et d'une plage de translation pour déplacer le rayon vers le haut ou vers le bas de l'axe.

12. Dispositif (1) selon la revendication 4,
dans lequel la circuiterie (2) est en outre configurée pour
- utiliser un modèle géométrique représenté par un maillage triangulaire pour identifier, pour un rayon respectif sortant de la surface de collecteur de LVOT, la coordonnée 3D où le rayon croise l'un des sommets des triangles du maillage ; et
- utiliser la coordonnée 3D comme point de départ d'un rayon qui estime l'épaisseur locale du septum.

13. Dispositif (1) selon l'une quelconque des revendications précédentes,
dans lequel la circuiterie (2) est en outre configurée pour visualiser les informations d'épaisseur de paroi déterminées et/ou le septum membraneux segmenté à la surface de ladite partie de la LVOT segmentée ou sur un reformatage aplati de ladite partie de la LVOT segmentée.

14. Procédé mis en œuvre par ordinateur (10) de segmentation du septum membraneux dans un ensemble de données d'image 3D, le procédé comprenant :
- la segmentation (11) de la cavité de chasse du ventricule gauche, LVOT, du cœur dans l'ensemble de données d'image 3D;
- la détermination (12) d'informations d'épaisseur de paroi indiquant l'épaisseur de la paroi du septum à différents endroits d'une partie de la LVOT segmentée qui est orientée vers la cavité cardiaque droite et l'oreillette droite ;
- le report (13) des informations d'épaisseur de paroi déterminées sur la surface de ladite partie de la LVOT segmentée ; et
- la segmentation (14) du septum membraneux dans l'ensemble de données d'image 3D sur la base des informations d'épaisseur de paroi reportées.

15. Programme informatique comprenant des moyens de code de programme pour amener un ordinateur à exécuter les étapes du procédé selon la revendication 14 lorsque ledit programme informatique est exécuté sur l'ordinateur.
